(19) Europäisches Patentamt
European Patent Office
Office européen des brevets

(11) Publication number : 0 431 659 B1

(12) EUROPEAN PATENT SPECIFICATION

(45) Date of publication of patent specification : 20.04.94 Bulletin 94/16

(51) Int. Cl.$^5$ : **A61K 31/12**, A61K 9/66, A61K 9/48, A61K 47/26

(21) Application number : 90203016.2

(22) Date of filing : 14.11.90

# (54) Pharmaceutical compositions of tebufelone.

The file contains technical information submitted after the application was filed and not included in this specification

(30) Priority : 22.11.89 US 440178

(43) Date of publication of application : 12.06.91 Bulletin 91/24

(45) Publication of the grant of the patent : 20.04.94 Bulletin 94/16

(84) Designated Contracting States : AT BE CH DE DK FR GB IT LI LU NL SE

(56) References cited :
EP-A- 0 251 408
EP-A- 0 313 303

(56) References cited :
JOURNAL OF CHROMATOGRAPHY, vol. 505, 1990, pages 349-356, Elsevier Science Publishers B.V., Amsterdam, NL; R.M. KAFFENBERGER et al.: "Determination of tebufelone (a new anti-inflammatory drug) strength and stability in bulk drug, dosage formulations and feed admixtures by reversed-phase high-performance liquid chromatography"

(73) Proprietor : THE PROCTER & GAMBLE COMPANY
One Procter & Gamble Plaza
Cincinnati Ohio 45202 (US)

(72) Inventor : Kelm, Gary Robert
8524 Althaus Road
Cincinnatti, Ohio 45247 (US)
Inventor : Bruns, Alan Edwards
7059 Okeana-Drewsburg Road
Okeana, Ohio 45053 (US)

(74) Representative : Suslic, Lydia et al
Procter & Gamble European Technical Center N.V. Temselaan 100
B-1853 Strombeek-Bever (BE)

EP 0 431 659 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The subject invention is concerned with novel pharmaceutical compositions containing tebufelone, a di-tert-butylphenol anti-inflammatory compound. More particularly, it is concerned with such compositions dosed per orally which provide good bioavailability of the compound.

Certain substituted di-tert-butylphenol derivatives are known to be effective as anti-inflammatory, analgesic and/or antipyretic agents. Of particular interest regarding the subject invention is tebufelone, 1-[3,5-bis(1,1-dimethylethyl)-4-hydroxyphenyl]-5-hexyn-1-one, disclosed in U.S. Patent No. 4,708,966 issued to Loomans, Matthews &Miller on November 24, 1987. (The compound is termed 4-(5'-hexynoyl)-2,6-di-tert-butylphenol therein.) Related compounds are disclosed in U.S. Patent No. 4,847,303 issued to Loomans, Matthews & Miller on July 11, 1989 and U.S. Patent No. 4,849,428 issued to Dobson, Loomans, Matthews &Miller on July 18, 1989.

It is an object of the subject invention to provide pharmaceutical compositions for peroral administration of the above anti-inflammatory compound which provide good bioavailability of the compound.

The subject invention relates to compositions consisting essentially of the drug active 1-[3,5-bis(1,1-dimethylethyl)-4-hydroxyphenyl]-5-hexyn-1-one at a concentration of at least about 15%, and the balance a pharmaceutically-acceptable vehicle. The compositions have the properties of (1) being a homogeneous liquid at 37°C, (2) providing solubilization of the drug active at a level of at least 1 mg/mL in 0.1 N HCl at 20°C, and (3) providing solubilization of the drug active in simulated intestinal fluid in 5 minutes or less.

The drug active of interest regarding the subject invention is 1-[3,5-bis(1,1-dimethylethyl)-4-hydroxyphenyl]-5-hexyn-1-one having the chemical structure:

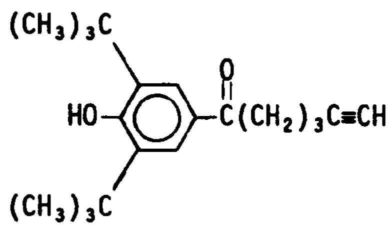

which is referred to herein as tebufelone. A method of synthesizing tebufelone is disclosed in aforementioned U.S. Patent No. 4,708,966, which is hereby incorporated herein by reference.

The subject invention involves pharmaceutical compositions of tebufelone intended for peroral administration to humans and lower animals. It has been found that tebufelone is essentially water-insoluble (solubility less than 1 μg/mL) and very lipophilic. The therapeutic dose of tebufelone is approximately 100 mg per day in humans. It has been found that the absorption of tebufelone from the gastrointestinal tract is quite low when tebufelone is dosed in conventional solid dosage forms, such as tablets or powders in capsules.

It has been found that good absorption of tebufelone from the gastrointestinal tract occurs only when tebufelone is perorally administered in pharmaceutical compositions which provide both rapid solubilization of tebufelone and also essentially its complete solubilization in the gastrointestinal fluids. As used herein, being solubilized means that tebufelone exists in an aqueous medium in a form that is freely diffusible. A freely diffusible form is one that is capable of transversing the unstirred boundary layer present along the absorbing membrane of the gastrointestinal tract. Such freely diffusible forms include a pure aqueous solution of tebufelone, an aqueous micellar solution of tebufelone (drug molecules dissolved in surfactant micelles), and/or an emulsion of tebufelone (liquid droplets containing drug active surrounded by a surfactant layer dispersed in an aqueous medium).

A composition of the subject invention consists essentially of tebufelone at a concentration of at least about 15%, and the balance a pharmaceutically-acceptable vehicle, the composition having the following properties:

(1) being a homogeneous liquid at 37°C,

(2) providing solubilization of tebufelone at a level of at least 1 mg/mL in 0.1 N HCl at 20°C, and

(3) providing solubilization of 20 mg of tebufelone in 500 mL of simulated intestinal fluid in 5 minutes or less.

As used herein, solubilization of a dispersion is considered to have occurred if substantially all of the dispersion will pass through a 0.45 μm filter. Whether the extent of solubilization of tebufelone meets requirement (2) above is determined by adding an amount of a composition containing 10 mg tebufelone to 10 mL 0.1 N HCl, shaking to disperse the composition, and determining whether substantially all of the dispersion will pass through a 0.45 μm filter.

The rate of solubilization of tebufelone of requirement (3) above is determined using the USP dissolution testing Apparatus 2 (see The United States Pharmacopia, XXth Revision (1979), p. 959). An amount of a composition containing 20 mg tebufelone is added to 500 mL simulated intestinal fluid, USP (without pancreatin), containing 2% of a 50/50 mixture of sodium cholate and sodium deoxycholate. Whether sufficient solubilization has occurred is determined after 5 minutes of stirring by determining whether substantially all of the dispersion passes through a 0.45 μm filter. An aliquot of the dispersion which has been filtered through a 0.45 μm filter is assayed for tebufelone by ultraviolet spectrophotometric assay.

One aspect of the subject invention involves compositions having a vehicle comprising a surfactant or mixture of surfactants, the vehicle having the following properties:

(a) being a homogeneous liquid at 37°C,
(b) having an HLB of from about 9 to about 13, and
(c) forming a stable dispersion in water at 20°C at concentrations of 10% or less.

Preferred vehicles also have the following properties:

(d) being soluble in isopropanol at 20°C at concentrations of 10% or less, and
(e) being soluble in cottonseed oil at 20°C at concentrations of 1% or less.

As used herein, HLB refers to the hydrophilic/lipophilic balance of the molecule as described in Griffin, W.C., "Classification of Surface-Active Agents by 'HLB'", Journal of the Society of Cosmetic Chemistry, Vol. 1, No. 5 (1949), p. 311. The HLB of the vehicle is preferably from 10 to 12.

Compositions of the subject invention comprise at least about 15% tebufelone, preferably from about 15% to about 30% tebufelone, more preferably from about 18% to about 25% tebufelone, especially from about 18% to about 20% tebufelone.

Preferred examples of surfactants which can be used in compositions of the subject invention include the following: polysorbate 80* and polysorbate 81* available from ICI Americas, Inc., Wilmington, Delaware; PEG-25 glyceryl trioleate* available from Goldschmidt Chemical Corp., Hopewell, Virginia; poloxamer 182*, poloxamer 183* and poloxamer 184* available from BASF Corp., Parsippany, New Jersey; and polyoxyl 35 castor oil** available from BASF Corp.; and mixtures thereof. (*See CTFA Cosmetic Ingredient Dictionary, Third Edition (1984), N.E. Estrin, P.A. Crosely & C.R. Haynes, Editors, The Cosmetic, Toiletry and Fragrance Association, Inc., Washington, D.C. **See The National Formulary, 17th Edition (1990), The United States Pharmacopeial Convention, Inc., Rockville, Maryland.)

Examples of surfactants which are preferably used as the sole surfactant in compositions of the subject invention include polysorbate 81, PEG-25 glyceryl trioleate, poloxamer 183 and poloxamer 184. Examples of surfactants which are preferably used as surfactant mixtures in compositions of the subject invention include the following: from about 25% to about 75% polyoxyl 35 castor oil and from about 75% to about 25% poloxamer 182, especially about 50% polyoxyl 35 castor oil and about 50% poloxamer 182; from about 10% to about 25% polysorbate 80 and from about 75% to about 90% poloxamer 182, especially about 17% polysorbate 80 and about 83% poloxamer 182.

The vehicle of the compositions of the subject invention may also comprise a lipophilic solvent for tebufelone. Preferred lipophilic solvents are triglycerides or mixtures of triglycerides having straight fatty chains which are saturated or unsaturated with from about 6 to about 10 carbon atoms. Other preferred lipophilic solvents are fatty acids or mixtures of fatty acids having saturated straight chains of from about 6 to about 10 carbon atoms, or unsaturated straight chains of from about 12 to about 18 carbon atoms. Preferred lipophilic solvents useful in compositions of the subject invention include caprylic/capric triglyceride* (Captex 300®), available from Capitol City Products Co., Columbus, Ohio; oleic acid; and linoleic acid.

Preferred compositions of the subject invention which have vehicles which are mixtures of lipophilic solvents and surfactants have vehicles comprising, preferably consisting essentially of, from about 25% to about 85% of the lipophilic solvent, and from about 15% to about 75% of the surfactant; more preferably from about 40% to about 70% of the lipophilic solvent, and from about 30% to about 60% of the surfactant.

Preferred examples of vehicles which are mixtures of lipophillc solvents and surfactants include the following: from about 20% to about 50% polysorbate 81 and from about 50% to about 80% caprylic/capric triglyceride, especially about 33% polysorbate 81 and about 67% caprylic/capric triglyceride; from about 2% to about 10% polyoxyl 35 castor oil, from about 15% to about 40% poloxamer 182, and from about 50% to about 83% caprylic/capric triglyceride, especially about 6% polyoxyl 35 castor oil, about 28% poloxamer 182 and about 66% caprylic/capric triglyceride; from about 10% to about 25% poloxamer 182, from about 10% to about 25% polyoxyl 35 castor oil, and from about 50% to about 80% oleic acid, especially about 17% poloxamer 182, about 17% polyoxyl 35 castor oil and about 66% oleic acid.

Especially preferred compositions of the subject invention consist essentially of the following combinations of components:

(a) from about 15% to about 20% tebufelone, from about 35% to about 45% polyoxyl 35 castor oil, and

from about 35% to about 45% poloxamer 182;

(b) from about 15% to about 20% tebufelone, from about 12% to about 16% polysorbate 80, and from about 64% to about 73% poloxamer 182;

(c) from about 15% to about 20% tebufelone, from about 25% to about 30% polysorbate 81, and from about 50% to about 60% caprylic/capric triglyceride;

(d) from about 15% to about 20% tebufelone, from about 4% to about 6% polyoxyl 35 castor oil, from about 20% to about 25% poloxamer 182, and from about 50% to about 60% caprylic/capric triglyceride;

(e) from about 15% to about 20% tebufelone, from about 12% to about 16% poloxamer 182, from about 12% to about 16% polyoxyl 35 castor oil, and from about 50% to about 60% oleic acid.

Another aspect of the subject invention involves compositions having a vehicle comprising triglycerides interesterified with polyethylene glycol. These materials are liquid at 37°C and have an HLB in the range of from about 3 to about 7, preferably from 5 to 7.

Preferred examples of such materials are glycolysed ethoxylated glycerides obtained by partial alcoholysis of natural vegetable oils, e.g., those available under the trade name Labrafil® from Gattefosse Corp., Elmsford, NY. A preferred example of such material is Labrafil 2609®, glycolysed ethoxylated glycerides obtained by partial alcoholysis of corn oil with polyethylene glycol 400.

Preferred compositions of the subject invention consist essentially of tebufelone, preferably in the amounts listed above, and the balance a glycolysed ethoxylated glyceride. Especially preferred are compositions consisting essentially of from about 15% to about 20% tebufelone and from about 80% to about 85% glycolysed ethoxylated glyceride.

## EXAMPLES

The following are non-limiting examples of compositions of the subject invention. The compositions are each made as follows:

(1) If a surfactant or lipid solvent component is a solid at room temperature, it is heated to melting;

(2) all components are mixed thoroughly until the tebufelone is dissolved in the composition.

### Example 1

| Component | Wt. % |
|---|---|
| Polyoxyl 35 Caster Oil | 41.0 |
| Poloxamer 182 | 41.0 |
| Tebufelone | 18.0 |

### Example 2

| Component | Wt. % |
|---|---|
| Polyoxyl 35 Caster Oil | 13.7 |
| Poloxamer 182 | 13.7 |
| Oleic Acid | 54.6 |
| Tebufelone | 18.0 |

### Example 3

| Component | Wt. % |
|---|---|
| Polysorbate 80 | 13.7 |
| Poloxamer 182 | 68.3 |
| Tebufelone | 18.0 |

Example 4

| Component | Wt. % |
|---|---|
| Polysorbate 80 | 4.6 |
| Poloxamer 182 | 22.8 |
| Captex 300® | 54.6 |
| Tebufelone | 18.0 |

Example 5

| Component | Wt. % |
|---|---|
| Polyoxyl 35 Castor Oil | 4.6 |
| Poloxamer 182 | 22.8 |
| Captex 300® | 54.6 |
| Tebufelone | 18.0 |

Example 6

| Component | Wt. % |
|---|---|
| Labrafil 2609® | 82.0 |
| Tebufelone | 18.0 |

Example 7

| Component | Wt. % |
|---|---|
| Polysorbate 81 | 27.4 |
| Captex 300® | 54.6 |
| Tebufelone | 18.0 |

Another aspect of the subject invention is unit dosage forms of compositions of the subject invention disclosed hereinabove. Compositions which are solid at room temperature can be reduced to a particulate form by conventional means such as by grinding, cutting or chopping, or by cooling a spray of liquid composition to form solid particles. Such particulate solids can be filled into hard gelatin capsule shells by conventional means. In addition, such compositions may be filled as hot liquids into hard gelatin capsule shells followed by cooling to allow the contents to solidify.

Preferred unit dosage forms of the subject invention are soft gelatin capsules or sealed hard gelatin capsules containing liquid or solidified compositions of the subject invention as disclosed hereinabove. The soft gelatin capsules are made by conventional means by filling liquid compositions of the subject invention into soft gelatin capsule shells. This can be done with the composition at room temperature if it is a liquid thereat, or by heating the composition above its melting temperature to produce a liquid, and filling such liquid into soft gelatin capsule shells. In a similar manner, sealed hard gelatin capsules contining liquid or solidified compositions of the subject invention can be made by conventional means.

## Claims

1. A composition characterized in that it consists essentially of 1-[3,5-bis(1,1-dimethylethyl)-4-hydroxyphenyl]-5-hexyn-1-one (tebufelone) at a concentration of a least 15%, preferably from 15% to 30, and the balance a pharmaceutically-acceptable vehicle, the composition having the following properties:
(1) being a homogeneous liquid at 37°C,
(2) providing solubilization of tebufelone at a level of at least 1 mg/mL in 0.1 HCl at 20°C, and
(3) providing solubilization of 20 mg of tebufelone in 500 mL of simulated intestinal fluid in 5 minutes or less;
the vehicle comprising, preferably consisting essentially of, a surfactant or mixture of surfactants, the vehicle having the following properties:
(a) being a homogeneous liquid at 37°C,
(b) having an HLB of from 9 to 13,
(c) forming a stable dispersion in water at 20°C at concentrations of 10% or less;
the vehicle additionally preferably having the following properties:
(d) being soluble in isopropanol at 20°C at concentrations of 10% or less, and
(e) being soluble in cottonseed oil at 20°C at concentrations of 1% or less.

**2.** The composition of Claim 1 wherein the surfactant is selected from polysorbate 80, polysorbate 81, poloxamer 182, poloxamer 183, poloxamer 184, PEG-25 glyceryl trioleate, and polyoxyl 35 castor oil, and mixtures thereof.

**3.** The composition of Claim 1 wherein the surfactant is selected from 100% polysorbate 81, 100% PEG-25 glyceryl trioleate, 100% poloxamer 183, 100% poloxamer 184, from 25% to 75% polyoxyl 35 castor oil and from 25% to 75% poloxamer 182, and from 10% to 25% polysorbate 80 and from 75% to 90% poloxamer 182.

**4.** The composition of Claims 1-3 wherein the vehicle also comprises a lipophilic solvent selected from a triglyceride or a mixture of triglycerides having fatty chains of from 6 to 10 carbon atoms, straight-chain saturated fatty acids having from 6 to 10 carbon atoms, and straight-chain unsaturated fatty acids having from 12 to 18 carbon atoms, and mixtures thereof.

**5.** The composition of Claims 1-4 wherein the vehicle consists essentially of from 25% to 85% of a lipophilic solvent which is selected from caprylic/capric triglyceride, oleic acid and linoleic acid.

**6.** The composition of Claim 1 consisting essentially of from 15% to 20% 1-[3,5-bis-(1,1-dimethylethyl)-4-hydroxyphenyl]-5 hexyn-1-one, from 4% to 6% polyoxyl 35 castor oil, from 20% to 25% poloxamer 182, and from 50% to 60% caprylic/-capric triglyceride.

**7.** A composition characterized in that it consists essentially of 1-[3,5-bis(1,1-dimethylethyl)-4-hydroxyphenyl]-5-hexyn-1-one (tebufelone) at at concentration of at least 15% preferably 15% to 30, and the balance a pharmaceutically-acceptable vehicle, the composition having the following properties:
(1) being a homogeneous liquid at 37°C,
(2) providing solubilization of tebufelone of at least 1 mg/mL in 0.1 N HCl at 20°C, and
(3) providing solubilization of 20 mg of tebufelone in 500 mL of simulated intestinal fluid in 5 minutes or less;
the vehicle comprising, preferably consisting essentially of, triglycerides interesterified with polyethylene glycol, such that its HLB is from 3 to 7.

**8.** The composition of Claim 7 wherein the triglycerides interesterified with polyethylene glycol are glycolysed ethoxylated glycerides obtained by partial hydrolysis of natural vegetable oils, preferably the triglycerides interesterified with polyethylene glycol are glycolysed ethoxylated glycerides obtained by partial hydrolysis of corn oil with polyethylene glycol 400.

**9.** A pharmaceutical unit dosage form characterized in that it comprises a composition of any of Claims 1-8 in a soft gelatin capsule shell.

**10.** A pharmaceutical unit dosage form characterized in that it comprises a composition of any of Claims 1-8 in a sealed hard gelatin capsule shell.

## Patentansprüche

1. Zusammensetzung, dadurch gekennzeichnet, daß sie im wesentlichen aus 1-[3,5-Bis-(1,1-dimethylethyl)-4-hydroxyphenyl]-5--hexin-1-on (Tebufelon) in einer Konzentration von wenigstens 15%, vorzugsweise von 15% bis 30%, besteht, wobei der Rest ein pharmazeutisch annehmbares Vehikel ist, und wobei die Zusammensetzung die folgenden Eigenschaften aufweist:
   (1) daß sie bei 37°C eine homogene Flüssigkeit ist;
   (2) daß sie eine Solubilisierung des Tebufelons in einer Menge von wenigsens 1 mg/ml in 0,1N HCl bei 20°C ergibt, und
   (3) daß sie eine Solubilisierung von 20 mg Tebufelon in 500 ml von simulierter Darmflüssigkeit in 5 min oder darunter ergibt;
   
   und daß Vehikel ein grenzflächenaktives Mittel oder ein Gemisch von grenzflächenaktiven Mitteln enthält oder vorzugsweise im wesentlichen aus einem solchen besteht, und daß das Vehikel die folgenden Eigenschaften aufweist:
   (a) daß es bei 37°C eine homogene Flüssigkeit ist;
   (b) daß es einen HLB-Wert von 9 bis 13 aufweist;
   (c) daß es in Wasser bei 20°C und bei Konzentrationen von 10% oder darunter eine stabile Dispersion bildet;
   
   und daß das Vehikel zusätzlich vorzugsweise auch noch die folgenden Eigenschaften aufweist:
   (d) daß es in Isopropanol bei 20°C und bei Konzentrationen von 10% oder darunter löslich ist; und
   (e) daß es in Baumwollsamenöl bei 20°C und bei Konzentrationen von 1% oder darunter löslich ist.

2. Zusammensetzung nach Anspruch 1, wobei das grenzflächenaktive Mittel aus Polysorbat-80, Polysorbat 81, Poloxamer 182, Poloxamer 183, Poloxamer 184, PEG-25-Glyceryltrioleat, Polyoxyl-35-Rizinusöl und Cemischen hievon ausgewählt ist.

3. Zusammensetzung nach Anspruch 1, wobei das grenzflächenaktive Mittel aus 100% Polysorbat 81, 100% PEG-25-Glyceryltrioleat, 100% Poloxamer 183, 100% Poloxamer 184, 25% bis 75% Polyoxyl-35-Rizinusol und 25% bis 75% Poloxamer 182, und aus 10% bis 25% Polysorbat 80 und 75% bis 90% Poloxamer 182 ausgewählt ist.

4. Zusammensetzung nach den Ansprüchen 1 bis 3, wobei das Vehikel auch ein lipophiles Lösungsmittel enthält, welches aus einem Triglycerid oder einem Gemisch von Triglyceriden mit Fettsäurekettenlängen von 6 bis 10 Kohlenstoffatornen, geradkettigen, gesättigten Fettsäuren mit 6 bis 10 Kohlenstoffatomen, geradkettigen, ungesättigten Fettsäuren mit 12 bis 18 Kohlenstoffatomen und Gemischen hievon ausgewählt ist.

5. Zusammensetzung nach den Ansprüchen 1 bis 4, wobei das Vehikel im wesentlichen zu 25% bis 85% aus einem lipophilen Lösungsmittel besteht, welches aus Capryl-/Caprinsäuretriglycerid, Ölsäure und Linolsäure ausgewählt ist.

6. Zusammensetzung nach Anspruch 1, welche im wesentlichen zu 15% bis 20% aus 1-[3,5-Bis-(1,1-dimethylethyl)-4-hydroxyphenyl]--5-hexin-1-on, zu 4% bis 6% aus Polyoxyl-35-Rizinusöl, zu 20% bis 25% aus Poloxamer 182, und zu 50% bis 60% aus Capryl-/Caprinsäuretriglycerid besteht.

7. Zusammensetzung, dadurch gekennzeichnet, daß sie im wesentlichen aus 1-[3,5-Bis-(1,1-dimethylethyl)-4-hydroxyphenyl]-5--hexin-1-on (Tebufelon) in einer Konzentration von wenigstens 15%, vorzugsweise von 15% bis 30%, besteht, wobei der Rest ein pharmazeutisch annehmbares Vehikel ist, und wobei die Zusammensetzung die folgenden Eigenschaften aufweist:
   (1) daß sie bei 37°C eine homogene Flüssigkeit ist;
   (2) daß sie eine Solubilisierung des Tebufelons von wenigstens 1 mg/ml in 0,1N HCl bei 20°C ergibt; und
   (3) daß sie eine Solubilisierung von 20 mg Tebufelon in 500 ml von simulierter Darmflüssigkeit in 5 min oder darunter ergibt;
   
   und daß das Vehikel mit Polyethylenglykol umveresterte Triglyceride enthält oder vorzugsweise im wesentlichen aus solchen besteht, derart, daß sein HLB-Wert 3 bis 7 beträgt.

8. Zusammensetzung nach Anspruch 7, wobei die mit Polyethylenglykol umveresterten Triglyceride glykolysierte, ethoxylierte Glyceride sind, welche durch eine partielle Hydrolyse von natürlichen Pflanzenölen erhalten worden sind, und wobei Vorzugsweise die mit Polyethylenglykol umveresterten Triglyceride glykolysierte, ethoxylierte Glyceride sind, welche durch partielle Hydrolyse von Maisöl mit Polyethylenglykol 400 erhalten worden sind.

**9.** Pharmazeutische Einheitsdosierungsform, dadurch gekennzeichnet, daß sie eine Zusammensetzung nach einem der Ansprüche 1 bis 8 in einer Weichgelatinekapselschale enthält.

**10.** Pharmazeutische Einheitsdosierungsform, dadurch gekennzeichnet, daß sie eine Zusammensetzung nach einem der Ansprüche 1 bis 8 in einer versiegelten Hartgelatinekapselschale enthält.

## Revendications

**1.** Composition caractérisée en ce qu'elle est constituée essentiellement de 1-[3,5-bis(1,1-diméthyléthyl)-4-hydroxyphényl]-5-hexyne-1-one (tébufélone) à une concentration d'au moins 15 % et de préférence de 15 à 30 %, le reste étant constitué d'un véhicule pharmaceutiquement acceptable, la composition ayant les propriétés suivantes :

(1) il s'agit d'un liquide homogène à 37°C,

(2) elle assure une solubilisation de la tébufélone a une concentration d'au moins 1 mg/ml dans du HCl 0,1 N à 20°C, et

(3) elle assure une solubilisation de 20 mg de tébufélone dans 500 ml de suc intestinal simulé, en 5 minutes ou moins :

le véhicule comprenant ou de préférence étant essentiellement constitué d'un tensioactif ou d'un mélange de tensioactifs, le véhicule ayant les propriétés suivantes :

(a) il s'agit d'un liquide homogène à 37°C,

(b) il présente un rapport hydro-lipophile de 9 à 13,

(c) il forme une dispersion stable dans l'eau à 20°C à des concentrations de 10 % ou moins,

le véhicule ayant de préférence en outre les propriétés suivantes :

(d) il est soluble dans l'isopropanol à 20°C à une concentration de 10 % ou moins, et

(e) il est soluble dans l'huile de graine de coton à 20°C à une concentration de 1 % ou moins.

**2.** Composition selon la revendication 1, dans laquelle le tensioactif est choisi parmi le polysorbate 80, le polysorbate 81, le poloxamère 182. le poloxamère 183, le poloxamère 184, le trioléate de glycéryle PEG-25 et l'huile de ricin polyéthoxylée à 35 moles d'oxyde d'éthylène, et leurs mélanges.

**3.** Composition selon la revendication 1, dans laquelle le tensioactif est choisi parmi 100 % de polysorbate 81, 100 % de trioleate de glycéryle PEG-25, 100 % de poloxamère 183, 100 % de poloxamère 184, de 25 à 75 % d'huile de ricin polyéthoxylée à 35 moles d'oxyde d'éthylène, et de 25 à 75 % de poloxamère 182 et de 10 à 25 % de polysorbate 80 et de 75 à 90 % de poloxamère 182.

**4.** Composition selon les revendications 1 à 3, dans laquelle le véhicule comprend aussi un solvant lipophile choisi parmi les triglycérides ou les mélanges de triglycérides ayant des chaînes grasses ayant de 6 à 10 atomes de carbone, les acides gras saturés à chaîne droite ayant de 6 à 10 atomes de carbone et les acides gras insaturés a chaîne droite ayant de 12 à 18 atomes de carbone et leurs mélanges.

**5.** Composition selon les revendications 1 a 4, dans laquelle le véhicule est constitué essentiellement de 25 à 85 % d'un solvant lipophile choisi parmi les triglycérides caprylique/caprique, l'acide oléique et l'acide linoléique.

**6.** Composition selon la revendication 1, constituée essentiellement de 15 à 20 % de 1-[3,5-bis(1,1-diméthyléthyl)-4-hydroxyphényl]-5-hexyne-1-one, de 4 à 6 % d'huile de ricin polyéthoxylée à 35 moles d'oxyde d'éthylène, de 20 à 25 % de poloxamère 182 et de 50 à 60 % de triglycéride caprylique/caprique.

**7.** Composition caractérisée en ce qu'elle est constituée essentiellement de 1-[3,5-bis(1,1-diméthyléthyl)-4-hydroxyphényl)-5-hexyne-1-one (tébufélone), à une concentration d'au moins 15 % et de préférence de 15 à 30 %, le reste étant un véhicule pharmaceutiquement acceptable, la composition ayant les propriétés suivantes :

(1) il s'agit d'un liquide homogène à 37°C,

(2) elle assure une solubilisation de la tébufélone à une concentration d'au moins 1 mg/ml dans du HCl

0,1 N à 20°C, et

(3) elle assure une solubilisation de 20 mg de tébufélone dans 500 ml de suc intestinal simulé, en 5 minutes ou moins ;

le véhicule comprenant des triglycérides interestérifiés avec un polyéthylèneglycol, ou étant essentiellement constitué de ce dernier de façon que son rapport hydro-lipophile soit de 3 à 7.

**8.** Composition selon la revendication 7, dans laquelle les triglycérides interestérifiés avec le polyéthylèneglycol sont des glycérides éthoxylés glycolysés obtenus par hydrolyse partielle d'huiles végétales naturelles, et de préférence les triglycérides interestérifiés avec le polyéthylèneglycol sont des glycérides éthoxylés glycolysés obtenus par hydrolyse partielle d'huile de maïs avec du polyéthylèneglycol 400.

**9.** Forme posologique unitaire caractérisée en ce qu'elle comprend une composition selon l'une quelconque des revendications 1 à 8 dans une coque de capsule en gélatine molle.

**10.** Forme posologique unitaire, caractérisée en ce qu'elle comprend une composition selon l'une quelconque des revendications 1 à 8, dans une coque d'une gélule en gélatine dure fermée.